# EUROPÄISCHE PATENTSCHRIFT

(11) **EP 0 926 245 B1**
(45) Veröffentlichungstag und Bekanntmachung des Hinweises auf die Patenterteilung: **22.09.2004**
(21) Anmeldenummer: 98124326.4
(22) Anmeldetag: 21.12.1998
(51) Int. Cl.: C12Q 1/68

(54) **Nachweis von Harnblasenkarzinom in einer Urinprobe**
Method for detection of carcinoma of the urinary bladder within a urine sample
Procédé pour la détection d'un carcinome de la vessie dans un échantillon d'urine

(30) Priorität: 22.12.1997 DE 19757300
(43) Veröffentlichungstag der Anmeldung: 30.06.1999
(73) Patentinhaber: Roche Diagnostics GmbH, 68305 Mannheim-Waldhof (DE)
(72) Erfinder: Emrich, Thomas, Dr., 82393 Iffeldorf (DE)
(74) Vertreter: Weiss, Wolfgang, Dipl.-Chem. Dr.

(56) Entgegenhaltungen:
- WO-A-96/01835
- WO-A-96/25949
- WO-A-97/18322
- WO-A-97/35871
- KINOSHITA HIDEFUMI ET AL: "Detection of telomerase activity in exfoliated cells in urine from patients with bladder cancer." JOURNAL OF THE NATIONAL CANCER INSTITUTE (BETHESDA), Bd. 89, Nr. 10, 21. Mai 1997 (1997-05-21), Seiten 724-730, XP001094967 ISSN: 0027-8874
- YOSHIDA KAZUHIRO ET AL: "Telomerase activity in bladder carcinoma and its implication for noninvasive diagnosis by detection of exfoliated cancer cells in urine." CANCER, Bd. 79, Nr. 2, 15. Januar 1997 (1997-01-15), Seiten 362-369, XP001094969 ISSN: 0008-543X
- MÜLLER M ET AL: "Comparison of human telomerase RNA and telomerase activity in urine for diagnosis of bladder cancer." CLINICAL CANCER RESEARCH: AN OFFICIAL JOURNAL OF THE AMERICAN ASSOCIATION FOR CANCER RESEARCH. UNITED STATES AUG 1998, Bd. 4, Nr. 8, August 1998 (1998-08), Seiten 1949-1954, XP001093807 ISSN: 1078-0432

## Beschreibung

Die vorliegende Erfindung betrifft ein Verfahren zum Nachweis von Harnblasenkarzinomzellen in einer Urinprobe sowie dafür geeignete Reagenzien.

Das Urothelkarzinom der Harnblase ist der häufigste maligne Tumor des Harntrakts und tritt bei ca. 15 aus 100.000 Personen auf. Der Nachweis von Harnblasenkarzinomen erfordert derzeit invasive cytoskopische Untersuchungen. Ein zuverlässiges nichtinvasives Verfahren zum Nachweis von Urothelkarzinomen der Harnblase würde sowohl bei der Diagnose als auch bei der Nachsorge von Patienten mit Harnblasenkarzinomen neue Möglichkeiten eröffnen. Weiterhin wäre ein solches Verfahren zum Screening von Personen aus Risikogruppen wie etwa Rauchern oder Arbeitern in der chemischen Industrie von Nutzen.

Die humane Telomerase ist ein Ribonukleoprotein, das eine de novo Synthese von Telomerenden bewirken kann. Telomere sind spezifische Strukturen an den Enden von Chromosomen und bestehen in eukaryontischen Organismen aus vielen Repetitionen kurzer Sequenzen, z.B. der Sequenz TTA GGG beim Menschen. In somatischen Zellen führt jede Replikation der Zellen unweigerlich zu einer Verkürzung der Telomerenden und ab einem bestimmten Unterschreiten der Telomerlänge schließlich zum Absterben der Zelle.

Virustransformierte oder immortalisierte Zellen wie etwa Tumorzellen zeigen dagegen keine Reduzierung in der Telomerlänge. Dies ist durch die Aktivität der Telomerase begründet. Da die Expression der Telomerase nach bisherigen Erkenntnissen auf Tumorzellen, Keimzellen und immortalisierte Zellen beschränkt ist, stellt sie einen vielversprechenden Parameter für eine Diagnostik von Tumoren sowie einen Angriffspunkt für eine Tumortherapie dar.

Die Struktur der RNA-Domäne des für Telomerase kodierenden Gens wurde vor kurzem in der Maus (Blasco et al., Science 269 (1995), 1267-1270) und im Menschen (Feng et al., Science 269 (1995), 1236-1241) bestimmt. Das Gen für humane Telomerase RNA (hTR) ist auf dem Chromosom 3 Abschnitt q 26.3 lokalisiert (Soder et al., Oncogene 14 (1997), 1013-1021; Parkinson et al., Eur. J. Cancer 33 (1997), 727-734). Die Proteindomänen der Telomerase aus verschiedenen niederen Eukaryonten sind ebenfalls bekannt (Collins et al., Cell 81 (1995), 677-686). Beim Menschen ist eine für ein homologes Protein kodierende mRNA, dessen Expression mit der Telomeraseaktivität korreliert, vor kurzem entdeckt worden (Nakamura et al., Science 277 (1997), 955-969; Meyerson et al., Cell 90 (1997), 785-795).

Die Bestimmung von Telomerase-RNA zur Quantifizierung von Tumorzellen in einer Körperflüssigkeit wird in WO 97/18322 beschrieben. Verfahren zum Nachweis von Harnblasenkarzinom durch Bestimmung der Telomeraseaktivität sind bekannt (Müller et al., Int. J. Oncol. 9 (1996), 1169-1173; Yoshida et al., Cancer 79 (1997), 362-369; Lin et al., Clin. Cancer Res. 2 (1996), 929-932 und Kinoshita et al., J. Natl. Cancer. Inst. 89 (1997), 724-730; Dalbagni et al., Clinic. Cancer Res. (1997), 1593-1598; WO97/35871).

Lin et al. beschreiben den Nachweis der Telomeraseaktivität aus Gewebeproben von Harnblasenkarzinompatienten. Es wurde eine Sensitivität von 97,5% gefunden. Dieses Verfahren hat jedoch den Nachteil, daß die Probe nur durch invasive Maßnahmen gewonnen werden kann, so daß eine Anwendung in der klinischen Routine nicht in Betracht kommt.

Müller et al. beschreiben den Nachweis von Telomeraseaktivität in Gewebeproben und Blasenspülungen von Harnblasenkarzinompatienten. Im Urin von Harnblasenkarzinompatienten konnte keine Telomeraseaktivität nachgewiesen werden. Yoshida et al. beschreiben den Nachweis von Telomeraseaktivität im Urin von Harnblasenkarzinompatienten. Es wurde eine Testsensitivität von 62% gefunden. Kinoshita et al. beschreiben ebenfalls den Nachweis von Telomeraseaktivität in Urinproben, wobei eine Sensitivität von 55% gefunden wird. Dalbagni et al. beschreiben den Nachweis von Telomeraseaktivität in Urinproben. Die Sensitivität des Tests betrug 18 bis 50 % abhängig vom jeweiligen Tumorstadium.

WO97/35871 beschreibt ein Verfahren zum Nachweis von Harnblasenkarzinomzellen in einer Urinprobe durch Bestimmung der Telomeraseaktivität. Es wird eine Sensitivität von 87% bei einer direkten Weiterverarbeitung der Probe ohne Einfrieren beschrieben.

Angesichts dieses Standes der Technik wird ersichtlich, daß die Telomerase-Aktivität zwar ein potentiell nützlicher Parameter für den Nachweis von Harnblasenkarzinom ist, daß aber bisherige nichtinvasive Verfahren keine zuverlässigen Resultate liefern. Gerade beim Nachweis der Telomeraseaktivität in Urinproben werden bei verschiedenen Autoren stark voneinander abweichende Ergebnisse erhalten, was auf große Probleme hinsichtlich der Reproduzierbarkeit hindeutet.

Aufgabe der vorliegenden Erfindung war es somit, ein neues nichtinvasives Verfahren zum Nachweis von Harnblasenkarzinomen bereitzustellen, bei dem die Nachteile des Standes der Technik mindestens teilweise beseitigt sind. Insbesondere sollte das Verfahren eine hohe Sensitivität und Reproduzierbarkeit besitzen und darüber hinaus auch hinsichtlich Probengewinnung und -vorbereitung für routinemäßige diagnostische Anwendungen geeignet sein.

Diese Aufgabe wird gelöst durch ein Verfahren zum Nachweis von Harnblasenkarzinom in einer Urinprobe, wobei man
(a) eine Urinprobe bereitstellt und
(b) Telomerase-RNA-Komponente in der Probe bestimmt, wobei das Vorhandensein der Telomerase-RNA-Komponente in der Probe ein Indikator für das Vorliegen eines Harnblasenkarzinoms ist.

Überraschenderweise gelingt durch das erfindungsgemäße Verfahren ein sensitiver und reproduzierbarer Nachweis von Telomerase-RNA in einer so aggressiven Probe wie Urin, in der ein saurer pH-Wert, RNasen, Salze und Harnstoff vorliegen. Ein Vergleich des erfindungsgemäßen Verfahrens mit den aus dem Stand der Technik bekannten Verfahren zur Bestimmung der enzymatischen Telomerase-Aktivität zeigt, daß die erfindungsgemäße Bestimmung der Telomerase-RNA bei gleichartiger Probenvorbereitung signifikante Vorteile hinsichtlich der Sensitivität und Zuverlässigkeit aufweist.

Das Vorhandensein von Telomerase-RNA in der Probe ist ein Indikator für das Vorliegen eines Harnblasenkarzinoms, insbesondere wenn die Menge von Telomerase-RNA über einem vorbestimmten Schwellenwert liegt.

Schritt (a) des erfindungsgemäßen Verfahrens umfaßt das Bereitstellen einer Urinprobe. Diese Urinprobe stammt vorzugsweise aus einem Säugetier. Besonders bevorzugt handelt es sich um eine humane Urinprobe. Vorzugsweise umfaßt Schritt (a) die Isolierung von Zellen aus dem Urin, z.B. durch Zentrifugation, und deren Aufschluß. Dieser Aufschluß kann beispielsweise durch Detergens-Extraktion erfolgen, wobei die Zellen mit einem Extraktionspuffer behandelt werden, der 0,01 bis 5 Gew.-% eines nichtionischen oder zwitterionischen Detergens wie etwa CHAPS enthält. Andererseits kann der Aufschluß der Zellen auch eine RNA-Anreicherung, z.B. mit dem kommerziell erhältlichem RNAzol-Kit umfassen. Beide Aufschlußverfahren ergeben vergleichbare Resultate. Ebenfalls möglich ist ein Aufschluß der Zellen durch Hitzedenaturierung.

Der Aufschluß der Zellen wird vorzugsweise in Gegenwart eines RNase-Inhibitors wie etwa RNAsin durchgeführt. Nach dem Aufschluß kann die Probe eingefroren und zur Bestimmung wieder aufgetaut werden. Das Einfrieren erfolgt vorzugsweise durch Schockgefrieren in flüssigem Stickstoff. Nach dem Einfrieren ist die Probe für mehrere Wochen oder Monate bei der Temperatur von - 80°C stabil.

Ein Einfrieren der Probe ist jedoch nicht erforderlich. Überraschenderweise wurde gefunden, daß ein zuverlässiger Nachweis der Telomerase-RNA auch ohne Aufarbeitung des Urins nach mehrtägiger Aufbewahrung bei 4°C oder bei Raumtemperatur möglich ist. Dies ist ein unerwarteter Befund, da die enzymatische Aktivität der Telomerase-RNA in einer Urinprobe bereits nach einer nur sehr kurzen Aufbewahrungsdauer nicht mehr nachweisbar ist. Diese überraschende Probenstabilität ist eine Voraussetzung für die Routineanwendung in der Diagnostik, da hier die Probenentnahme und die Probenaufarbeitung üblicherweise an verschiedenen Orten durch verschiedene Personen erfolgt.

Schritt (b) des erfindungsgemäßen Verfahrens umfaßt die Bestimmung einer Telomerase-RNA, vorzugsweise die Bestimmung der humanen Telomerase-RNA. Diese Bestimmung kann ohne Markierungsgruppen, mit radioaktiven Markierungsgruppen oder mit nichtradioaktiven Markierungsgruppen erfolgen. Vorzugsweise werden nichtradioaktive Markierungsgruppen verwendet, die ausgewählt werden aus immunologisch reaktiven Gruppen wie etwa Nukleotidanaloga oder Haptenen, die mit einem Nachweisantikörper reagieren können, Enzymen wie etwa Peroxidase, Galactosidase oder alkalische Phosphatase, fluoreszierenden Gruppen oder lumineszierenden Grupppen wie etwa Chemilumenszenz- oder Elektrochemilumineszenz-Gruppen, Donor-Akzeptor-Systemen oder anderen Nachweisgruppen wie etwa NMR-aktive Markierungsgruppen oder elektronendichte Gruppen. Bevorzugt sind immunologisch reaktive Gruppen wie etwa Nukleotidanaloga, z.B. halogenderivatisierte Nukleotide wie etwa Br-dUTP oder mit organischen Resten, die mindestens ein C-Atom enthalten, derivatisierte Nukleotide wie etwa CH₃-dCTP, oder Haptene, wie Digoxigenin, Digoxin, Fluorescein etc., lumineszierende Gruppen, wie etwa Acridiniumester oder lumineszierende Metallkomplexe, z.B. Ruthenium-Komplexe, und fluoreszierende Gruppen wie etwa Fluorescein, und Donor-Akzeptor-Systeme wie etwa Fluoreszenz-Resonanz-Energietransfer-Systeme.

Die Bestimmung der Telomerase-RNA gemäß Schritt (b) des erfindungsgemäßen Verfahrens umfaßt vorzugsweise einen reversen Transkriptionsschritt, wobei die Telomerase-RNA in eine komplementäre DNA umgeschrieben wird. Weiterhin umfaßt die Bestimmung vorzugsweise einen Nukleinsäure-Amplifikationsschritt. Die Art des Amplifikationsschritts ist für das erfindungsgemäße Verfahren nicht kritisch. Typischerweise erfolgt die Amplifikation durch Zugabe eines geeigneten Enzyms, das eine Polymerisierung von Nukleinsäuren bewirken kann, z.B. einer Nukleinsäure-Polymerase oder einer Nukleinsäure-Ligase. Vorzugsweise verwendet man ein thermostabiles Enzym und führt die Amplifikation in mehreren Zyklen durch. Besonders bevorzugt ist das Enzym eine thermostabile DNA-Polymerase, z.B. Taq-Polymerase. Ein besonders bevorzugtes Amplifiktionsverfahren ist die Methode der Polymerase-Kettenreaktion (PCR). Am meisten bevorzugt umfaßt die erfindungsgemäße Bestimmung der humanen Telomerase-RNA eine Kombination von Reverser Transkription und PCR.

Vorzugsweise werden zur Amplifikation zwei Primer verwendet, die zur Telomerase-RNA oder einer daraus synthetisierten cDNA komplementär sind. Bei humaner Telomerase-RNA kann die Auswahl geeigneter Primersequenzen auf Basis der bei Feng et al., supra, angegebenen Nukleotidsequenz erfolgen. Beispielsweise kann man für die Nukleinsäureamplifikation ein Primerpaar verwenden ausgewählt aus:
(a) Nukleotiden mit den in SEQ ID NO. 1 und SEQ ID NO. 2 gezeigten Nukleotidsequenzen und
(b) Nukleotiden, die eine Teilsequenz der in SEQ ID NO. 1 und SEQ ID NO. 2 gezeigten Nukleotidsequenzen mit einer Länge von mindestens 5 Nukleotiden enthalten.

Der Nachweis der Telomerase-RNA durch das erfindungsgemäße Verfahren kann qualitativ oder quantitativ erfolgen. Ein Ansatz, welcher ein von Telomerase-RNA stammendes Amplifikationsprodukt insbesondere über einem vorbestimmten Schwellenwert liegend ergibt, wird als positiv eingestuft. Der Schwellenwert wird vorzugsweise so festgelegt, daß die gelegentlich bei Normalpatienten auftretenden schwachen Signale als negativ und die von Harnblasenkarzinompatienten stammenden starken Signale als positiv eingestuft werden. Ein quantitativer Nachweis der Telomerase-RNA kann einen Rückschluß auf den Differenzierungsgrad oder das Stadium von Karzinomen ermöglichen, da die Signalstärke mit der Aggresivität des Karzinoms zunimmt.

Einerseits kann der Nachweis eine Auftrennung des Reaktionsgemisches nach der Größe umfassen, z.B. eine Auftrennung in einem nichtdenaturierenden Agarosegel. Neben der Größe können auch andere Eigenschaften der Amplifikationsprodukte zur Unterscheidung herangezogen werden, z.B. Vorhandensein/Nichtvorhandensein einer Restriktionsenzymschnittstelle, Schmelztemperatur der Amplifikationsprodukte etc.

Alternativ können die durch das erfindungsgemäße Verfahren erhaltenen Amplifikationsprodukte auch ohne vorherige Auftrennung des Reaktionsansatzes analysiert werden, z.B. nach Immobilisierung an einer Festphase oder durch on-line-Detektion in einem homogenen Testformat. Als Festphase kann beispielsweise die Wand des Reaktionsgefäßes dienen. Außerdem können auch partikuläre Festphasen eingesetzt werden. Vorzugsweise wird die Festphase ausgewählt aus Mikrotiterplatten, Mikroreaktionsgefäßen, Membranen, Mikrochips, Biocore-Systemen und gegebenenfalls magnetischen Mikrobeads. Ein Vorteil der Immobilisierung besteht darin, daß eine große Zeitersparnis bei geringerem manuellen Aufwand erreicht wird. Weiterhin ist ein hoher Probendurchsatz und eine Automatisierung, z.B. für die Routineanalytik möglich.

Die Immobilisierung der Amplifikationsprodukte an der Festphase kann grundsätzlich nach jeder bekannten Methode erfolgen, z.B. durch adsorptive Bindung. Vorzugsweise erfolgt die Immobilisierung durch spezifische Wechselwirkungen, z.B. über Ankergruppen. Beispiele für geeignete Ankergruppen sind immunologisch reaktive Gruppen, die mit einem Festphase-gebundenem Antikörper reagieren können, oder andere Gruppen, die zu einer hochaffinen Bindung mit einem immobilisierten Partner in der Lage sind. Ein bevorzugtes Beispiel für eine Ankergruppe ist Biotin, das mit hoher Affinität an eine mit Avidin oder Streptavidin beschichtete Festphase binden kann.

Eine on-line-Detektion kann z.B. durch Donor-Akzeptor-Nachweissysteme oder durch Acridiniumester-Nachweisgruppen in einem homogenen Nachweisverfahren erfolgen. Ein entsprechendes Testformat ist bei Arnold Jr. et al. (Clin. Chem. 35 (1989), 1588-1594 beschrieben.

Die Einführung von Markierungs- oder Ankergruppen in das Amplifikationsprodukt kann in verschiedenen Stadien des erfindungsgemäßen Verfahrens erfolgen. So kann man beispielsweise einen oder mehreren Primer verwenden, welche bereits Markierungsgruppen oder/und Ankergruppen enthalten. Andererseits können markierte Nukleotide auch während der Amplifikation in das Amplifikationsprodukt eingeführt werden.

In einer bevorzugten Ausführung der vorliegenden Erfindung ist es jedoch gar nicht erforderlich, daß das Amplifikationsprodukt selbst eine Ankeroder/und Markierungsgruppe enthält. Diese Gruppen können auch in einer oder mehreren Hybridisierungssonden vorliegen, die in der Lage sind, eine unter Testbedingungen stabile Hybridisierung mit dem Amplifikationsprodukt einzugehen, um auf diese Weise eine spezifische Detektion zu ermöglichen. Eine Verankerung an der Festphase kann beispielsweise durch Zugabe einer oder mehrerer Fangsonden erreicht werden, die eine oder mehrere Ankergruppen tragen. Auf entsprechende Weise können auch Markierungssonden zum Nachweis des Amplifikationsprodukts eingesetzt werden. Beispiele für geeignete Hybridisierungssonden sind Oligonukleotide, die an ihrem 5'-oder 3'-Ende eine Anker- oder Markierungsgruppe enthalten. Andererseits können auch Nukleinsäureanaloga als Hybridisierungssonden eingesetzt werden, z. B peptidische Nukleinsäuren (Nielsen et al., Science 254 (1991), 1497-1500 und Dueholm et al., J. Org. Chem. 59 (1994), 5767-5773).

Der Nachweis der Telomerase-RNA erfolgt vorzugsweise über die im Amplifikationsprodukt enthaltenen Markierungsgruppen bzw. über die an das Amplifikationsprodukt gebundenen Markierungssonden auf an sich bekannte Weise. Vorzugsweise erfolgt der Nachweis bei Verwendung nichtradioaktiver Markierungsgruppen in automatisierten Meßvorrichtungen. Bevorzugt sind Meßvorrichtungen, in denen der Nachweis der Markierungsgruppendurchcolorimetrischeoder/undspektrophotometrische Methoden erfolgt, z.B. durch enzymatische Umsetzung eines Substrats bzw. durch Chemilumineszenz oder Fluoreszenz.

Vorzugsweise wird das erfindungsgemäße Verfahren so durchgeführt, daß in der Probe neben der Bestimmung von Telomerase-RNA eine Bestimmung einer von der Telomerase-RNA verschiedenen Standard-Nukleinsäure, insbesondere einer RNA als Standard, durchgeführt wird. Auch Nukleinsäuren-ähnliche Moleküle , z.B. Nukleinsäureanaloga können als Standard-Nukleinsäure verwendet werden.

Der Standard ist vorzugsweise eine vom jeweiligen Organismus konstitutiv exprimierte RNA, z.B. eine humane rRNA, insbesondere die 28S rRNA, die β2-Mikroglobulin mRNA oder die GAPDH-mRNA. Andererseits kann als Standard auch eine der Proben von außen, z.B. vor der Probenaufarbeitung oder vor der Amplifikation, zugesetzte Nukleinsäure, insbesondere eine RNA verwendet werden. Als Standard kann darüber hinaus eine modifizierte Telomerase-RNA verwendet werden, die durch eine oder mehrere Merkmale von der nachzuweisenden Telomerase-RNA unterscheidbar ist, beispielsweise durch eine Mutation der Sequenz, z.B. durch Insertion oder/und Deletion von einem oder mehreren Nukleotidbausteinen. Die Bestimmung des Standards wird vorzugsweise auf analoge Weise wie die Bestimmung der humanen Telomerase-RNA durchgeführt und kann z.B. eine Reverse Transkription oder/und eine Nukleinsäure-Amplifikation umfassen. Besonders bevorzugt wird der Standard unter Verwendung einer Kombination von Reverser Transkription und PCR bestimmt.

Wenn man die humane β2-Mikroglobulin RNA als Standard bestimmt, hat sich die Verwendung eines Primerpaares bei der Nukleinsäureamplifikation als geeignet erwiesen, welches ausgewählt ist aus:
(a) Nukleotiden mit den in SEQ ID NO. 3 und SEQ ID NO. 4 gezeigten Nukleotidsequenzen und
(b) Nukleotiden, die eine Teilsequenz der in SEQ ID NO. 3 und SEQ ID NO. 4 gezeigten Nukleotidsequenzen mit einer Länge von mindestens 5 Nukleotiden enthalten.

Das erfindungsgemäße Verfahren kann als Ein-Topf-Reaktion durchgeführt werden, insbesondere wenn der Nachweis der Telomerase-RNA und gegebenenfalls des Standards unter solchen Bedingungen durchgeführt wird, die in eine spezifische Detektion der Telomerase-RNA und - sofern vorhanden - des Standards ohne Abtrennung von Amplifikationsnebenprodukten erlauben.

Diese spezifische Detektion ist bei elektrophoretischer Auftrennung der Amplifikationsprodukte auf einfache Weise durch Bestimmung einer Bande mit einer charakteristischen Größe im Elektrophoresemedium möglich. Bei paralleler Bestimmung eines Standards wird in diesem Fall zweckmäßigerweise ein Primerpaar verwendet, welches bei Amplifikation ein Produkt mit einer Größe ergibt, die sich eindeutig von der Größe des humanen Telomerase-RNA-Amplifikationsprodukts unterscheiden läßt. Auf entsprechende Weise ist auch eine spezifische Detektion über andere Eigenschaften der Amplifikationsprodukte möglich.

Bei Analyse der Produkte des Amplifikationsgemisches ohne vorherige Auftrennung z.B. durch Immobilisierung oder durch on-line-Detektion kann die Unterscheidung zwischen humaner Telomerase-RNA, Standard und gegebenenfalls Amplifikationsnebenprodukten durch verschiedene Maßnahmen erfolgen, z.B. durch Verwendung unterschiedlicher Markierungen oder/und Ankergruppen (z.B. Primer, Fangsonden oder/und Markierungssonden mit jeweils unterschiedlichen Gruppen), durch Auswahl von Hybridisierungsbedingungen, die eine solche spezifische Detektion erlauben, oder/und durch Immobilisierung des Standards und des Telomerase-RNA-Amplifikationsprodukts an verschiedenen Orten der Festphase oder an verschiedenen Festphasen. Weiterhin kann die Spezifität durch Zusatz unmarkierter Oligonukleotide oder Nukleinsäureanaloga (Kompetitoren) verbessert werden, die komplementär zu den Primersequenzen sind, so daß diese Sequenzbereiche maskiert werden und die Hybridisierung mit der Fang- oder Detektionssonde spezifisch an internen Sequenzen der Amplifikationsprodukte erfolgt.

Noch ein weiterer Gegenstand der vorliegenden Erfindung ist die Verwendung eines Reagenzienkits zum Nachweis einer Telomerase-RNA-Komponente umfassend:
(a) einen Primer für die Reverse Transkription einer Telomerase-RNA-Komponente,
(b) Mittel zur reversen Transkription,
(c) ein Primerpaar zur Amplifikation einer durch Reverse Transkription aus der Telomerase-RNA-Komponente erhaltenen DNA,
(d) Mittel zur Nukleinsäureamplifikation und
(e) Markierungsgruppen, z.B. nichtradioaktive Markierungsgruppen in einem nichtinvasiven Verfahren zum Nachweis von Harnblasenkarzinomen.

Die Markierungsgruppen können in Form von entsprechend markierten Nukleosidtriphosphaten oder/und markierten Primern vorliegen. Andererseits können die Markierungsgruppen auch auf einer oder mehreren Markierungssonden vorliegen, die unter Testbedingungen mit dem Amplifikationsprodukt hybridisieren.

Weiterhin kann der Reagenzienkit Festphasenankergruppen und eine Festphase enthalten. Die Festphasenankergruppen sind vorzugsweise Biotin und die Festphase ist mit Streptavidin oder/und Avidin beschichtet. Die Festphasenankergruppen können in Form modifizierter Nukleosidtriphosphate bzw. auf einem Primer vorliegen. Andererseits können die Festphasenankergruppen auch auf einer Fangsonde vorliegen, die mit dem Amplifikationsprodukt hybridisiert.

Alternativ kann der Reagenzienkit Nachweisgruppen und gegebenenfalls Hybridisierungssonden enthalten, mit denen eine on-line-Detektion insbesondere in einem homogenen Testformat möglich ist.

Die Mittel zur Reversen Transkription und zur Nukleinsäureamplifikation umfassen vorzugsweise für den jeweiligen Zweck geeignete Enzyme, Nukleosidtrisphosphate und gegebenenenfalls Puffersubstanzen.

Darüber hinaus kann der erfindungsgemäße Reagenzienkit einen Primer für die Reverse Transkription einer von der Telomerase-RNA verschiedenen Standard-Nukleinsäure und ein Primerpaar zur Amplifikation einer durch Reverse Transkription einer aus dem Standard erhaltenen DNA enthalten.

In einer besonders bevorzugten Ausführungsform enthält der Reagenzienkit eine oder mehrere Hybridisierungssonden, z.B. Markierungs- oder/und Fangsonden, die Markierungs- oder/und Festphasenankergruppen aufweisen und mit dem Amplifikationsprodukt bzw. bei Verwendung eines Standards mit dessen Amplifikationsprodukten hybridisieren. Die Markierungs- und Fangsonden werden vorzugsweise aus Oligonukleotiden und Nukleinsäureanaloga, insbesondere peptidischen Nukleinsäuren, ausgewählt.

Darüber hinaus kann der Reagenzienkit auch positive oder/und negative Kontrollen sowie einen internen Standard zur Quantifizierung der Nachweisreaktion und entsprechende Mittel zum separaten Nachweis von Standard und Amplifikationsprodukt enthalten.

Weiterhin wird die Erfindung durch die nachfolgenden Beispiele und Sequenzprotokolle erläutert. Es zeigen:
- SEQ ID NO. 1 und 2: Amplifikationsprimer zur Bestimmung der humanen Telomerase-RNA,
- SEQ ID NO.3 und 4: Amplifikationsprimer zur Bestimmung der β2-Mikroglobulin mRNA als Standard und
- SEQ ID NO. 5 und 6: Primer zur Bestimmung der Telomerase-Aktivität.

### Beispiele

### 1. Gewinnung und Vorbehandlung von Urinproben

Proben von jeweils 100 ml des zweiten Morgenurins wurden von 30 Patienten mit bestätigtem Urothelkarzinom der Harnblase, 15 Patienten mit anderen urologischen Krankheiten (Urolithiasis, Harnweginfektionen), 3 Patienten im Rahmen der Nachsorge bei Harnblasenkarzinomen und 20 gesunden Freiwilligen gewonnen.

Alle Proben wurden innerhalb von 15 min weiterverarbeitet. Die Proben wurden in 2 Aliquots von jeweils 50 ml unterteilt. Beide Aliquots wurden zentrifugiert und zweimal mit PBS gewaschen, wonach bei einer Probe eine CHAPS-Extraktion und bei der anderen eine RNAzol Extraktion durchgeführt wurde.

Zur CHAPS-Extraktion wurden die aus dem zentrifugierten Urin gewonnenen Zellpellets unter Verwendung von 200 µl Lysispuffer (10 mM Tris-HCl pH 7,5, 1 mM MgCl₂, 1 mM EGTA, 0,1 mM Phenylmethylsulfonylfluorid, 5 mM β-Mercaptoethanol, 0,5 % CHAPS, 10 % Glycerin) gemäß dem Verfahren von Kim et al. (Science 266 (1994), 2011-2015) lysiert. Das Lysat wurde dann in flüssigem Stickstoff schockgefroren und bei -80°C aufbewahrt.

Die RNAzol Extraktion der aus dem zentrifugierten Urin gewonnen Pellets wurde gemäß den Vorschriften des Herstellers (WAK-Chemie-Medical GmbH, Bad Homburg, Deutschland) durchgeführt. Das Lysat wurde in flüssigem Stickstoff schockgefroren und bei -80°C aufbewahrt.

### 2. Telomerase-Nachweisverfahren

### 2.1 Nachweis der Telomerase-Aktivität (TRAP-Assay)

Der TRAP-Assay wurde gemäß dem vom Kim et al. (supra) beschriebenen Verfahren durchgeführt. Der Proteingehalt aus der Probe wurde unter Verwendung der Biuret Methode bestimmt. Es wurden 10µg Proteinkonzentrat, 20 mM Tris HCl (pH 8,3), 1,5 mM MgCl₂, 63 mM KCI, 0,0005% Tween-20, 1 mM EGTA, 50 µM Deoxynucleotidtriphosphate, 0,1 µg Oligonukleotid TS (SEQ ID NO 5: 5'-AAT CCG TCG AGC AGA GTT-3'), 1 µg T4g32 Protein (Boehringer Mannheim) und 0, 1 mg/ml Rinderserumalbumin für 30 min bei 35°C inkubiert. Als negative Kontrollen wurden mit 1 µg RNAse inkubierte Proben sowie Leerproben mit Lysepuffer oder H₂O untersucht.

Für die Amplifikation wurden 2,5 U Taq-DNA Polymerase (Perkin-Elmer, Weiterstadt, Deutschland) und 0,1 µg Oligonukleotid Cx (SEQ ID NO. 6: 3'-AAT CCC ATT CCC ATT CCC ATT CCC-5') zu 50 µl Probenvolumen gegeben. Es wurden 30 PCR-Zyklen (94°C für 30 s, 50°C für 30 s und 72°C für 90 s) in einem Thermocycler (TC9600, Perkin-Elmer, Weiterstadt, Deutschland) durchgeführt.

Der Nachweis der PCR-Produkte erfolgte durch nichtradioaktive Markierung. Hierzu wurde der Cx-Primer mit dem Fluoreszenzfarbstoff FAM (Perkin-Elmer, Weiterstadt, Deutschland) markiert. Fluoreszente Amplifikationsprodukte wurden nach Elektrophorese in einem 4,5% Polyacrylamid/6 M Harnstoff-Sequenziergel auf einem automatisierten Sequenator 377 unter Verwendung des GeneScan Software-Pakets (Perkin-Elmer, Weiterstadt, Deutschland) nachgewiesen.

Parallel hierzu wurde der Telomerase PCR-ELISA-Kit (Boehringer Mannheim, Mannheim, Deutschland) zum Nachweis nichtradioaktiver PCR-Produkte unter Verwendung eines Biotin-markierten TS-Primers verwendet.

### 2.2 Nachweis humaner Telomerase-RNA (hTR) durch RT-PCR

Zur Durchführung der RT-PCR wurden 2 µg Gesamt-RNA aus der RNAzol-Extraktion oder 2 µg Gesamtprotein aus der CHAPS-Extraktion verwendet. Die PCR wurde nach Zugabe von 23 µl eines Reaktionsgemisches durchgeführt, welches die Reagenzien des Titan™ One Tube RT PCR Systems (Boehringer Mannheim) plus jeweils 150 µM der im folgenden angegebenen Primerpaare enthielt. Nach einem Denaturierungsschritt (6 min bei 94°C) und reverser Transkription (30 min bei 65°C) folgte ein weiterer kurzer Denaturierungsschritt und 25 Zyklen einer PCR (94°C für 30 s und 65°C für 1 min, Extension: 60°C für 7 min).

Zum Nachweis der Telomerase RNA wurden das Primerpaar TE-hTR 5.3 (SEQ ID NO. 1: 5'-AAC TGA GAA GGG CGT AGG CGC CGT GC-3') und TE-hTR 3.1 (SEQ ID NO. 2: 5'-GTT TGC TCT AGA ATG AAC GGT GGA AG-3') verwendet, mit denen ein 111 bp langes Amplifikationsprodukt erhalten wird. Als interner Standard wurde das Primerpaar TE-β2M 5.2 (SEQ ID NO. 3: 5'-TTC ACC CCC ACT GAA AAA GAT GA-3') und (SEQ ID NO. 4: 5'-GGC ATC TTC AAA CCT CCA TGA TG-3') zum Nachweis von β2-Mikroglobulin mRNA verwendet, mit dem ein Produkt von 119 bp entsteht. Zum nichtradioaktiven Nachweis der PCR-Produkte war jeweils einer der Primer (TE-hTR5.3 oder TE-β2M 5.2) mit FAM (Firma Perkin-Elmer) markiert. Elektrophorese und Nachweis der PCR-Produkte erfolgten wie unter Punkt 2.1 beschrieben.

Als positive Kontrollen wurden humane Gewebeproben aus einem Harnblasenkarzinom und Proben aus Zellen der humanen Harnblasenkarzinomzelllinie J82 (ATCC HTB1) verwendet. Diese Zellinie wurde in DMEM mit 10% fötalem Kälberserum, 2% L-Glutamin, 1 % Penicillin-Streptomycin (Gibco) kultiviert.

### 3. Ergebnisse

In J82 Zellen und Gewebeproben aus einem Blasenkarzinom konnten große Mengen an humaner Telomerase RNA (hTR) sowohl in CHAPS Lysat als auch in durch RNAzol-Extraktion erhaltenen RNA-Präparationen nachgewiesen werden. In diesen Proben wurde auch eine hohe Telomeraseaktivität gefunden. Nur Proben mit eindeutigen Banden von 119 bp (Anwesenheit der β2-Mikroglobulin mRNA) und 111 bp (Anwesenheit von hTR) wurden als positiv hinsichtlich der Bestimmung von hTR eingestuft. Proben mit einer Bande bei 119 bp und dem Fehlen einer Bande von 111 bp (Abwesenheit von hTR) wurden als negativ eingestuft.

Der TRAP-Assay von Telomerase-positiven Proben zeigte die charakteristische 6 bp Leiter nach GeneScan-Analyse. Beim Telomerase PCR ELISA zeigten Telomerase-positive Proben Absorptionen (A₄₅₀-A₆₉₀) zwischen 250 und 2.000. Mit RNase behandelte Proben zeigten keine Telomeraseaktivität. Leerproben (Lysepuffer oder Wasser) zeigten ebenfalls keine Telomeraseaktivität. Im Telomerase-PCR ELISA zeigten Telomerase-negative Proben und Kontrollen eine Absorption von weniger als 100. Beide Methoden zum Nachweis von TRAP-Produkten (GeneScan Analyse oder Telomerase PCR ELISA) ergaben vergleichbare Ergebnisse.

Nachweisbare Mengen von hTR wurden mit RT-PCR in 25 von 30 Urinproben (82%) gefunden, die von Patienten mit bestätigtem Harnblasenkarzinom stammten. Telomeraseaktivität wurde nur in zwei Fällen (7%) nachgewiesen. Bei gesunden Freiwilligen konnte durch RT-PCR die hTR in 3 von 20 Proben (15%) nachgewiesen werden.

Bei Patienten ohne maligner Erkrankung, aber mit bekannten benignen urologischen Erkrankungen, wie etwa Urolithiasis oder Harnwegsinfektionen, wurde hTR in 4 von insgesamt 15 Proben (27%) gefunden. Bei einer Patientin mit Harntraktinfektion und anfänglich nachweisbarer hTR wurde nach Behandlung der Infektion keine hTR mehr gefunden. Telomeraseaktivität konnte bei keinem Patienten nachgewiesen werden.

Proben von drei Patienten wurden im Rahmen der Nachsorge bei Harnblasenkarzinomen untersucht. Bei diesen Patienten, bei denen kein erneutes Auftreten der malignen Erkrankung festgestellt werden konnte, wurde in keinem Fall hTR oder Telomeraseaktivität nachgewiesen.

Die Ergebnisse sind in den folgenden Tabellen zusammengefaßt.

Tabelle 1 zeigt die klinisch pathologische Klassifizierung und die Ergebnisse des Nachweises von hTR und Telomeraseaktivität in Urinproben von Patienten mit histologisch bestätigtem Harnblasenkarzinom.

**Tabelle 1**

| **Patient** | **Stadium** | **Grad** | **Telomerase- Aktivität** | **hTR** |
|---|---|---|---|---|
| 1 | pTa | G1 | - | + |
| 2 | pTa | G1 | - | + |
| 3 | pTa | G2 | - | - |
| 4 | pT1 | G3 | + | + |
| 5 | pTa | G2 | - | + |
| 6 | pT3 | G3 | - | + |
| 7 | PT2 | G3 | - | + |
| 8 | pT1 | G2 | - | - |
| 9 | pT2 | G3 | - | + |
| 10 | pTa | G2 | + | + |
| 11 | pT1 | G2 | - | 1 |
| 12 | pTa | G2 | - | + |
| 13 | PT1 | G3 | - | - |
| 14 | pTa | G2 | - | - |
| 15 | pT1 | G3 | - | + |
| 16 | pT1 | G3 | - | + |
| 17 | pT2 | G3 | - | + |
| 18 | pTa | G2 | - | - |
| 19 | pT1 | G2 | - | + |
| 20 | PT1 | G3 | - | + |
| 21 | pT2 | G3 | - | + |
| 22 | PT2 | G3 | - | + |
| 23 | pTa | G2 | - | + |
| 24 | pTa | G2 | - | + |
| 25 | pT2 | G3 | - | + |
| 26 | pT3 | G3 | - | + |
| 27 | pTa | G1 | - | + |
| 28 | pTa | G2 | - | + |
| 29 | pT2 | G2 | - | + |
| 30 | pT2 | G3 | - | + |

Tabelle 2 zeigt eine Zusammenstellung der Ergebnisse beim Nachweis von hTR im Urin von Patienten mit Blasenkarzinom, benignen urologischen Erkrankungen und gesunden Personen.

**Tabelle 2**

| **Probe** | **hTR positive Proben** | **hTR negative Proben** | **Gesamt** | **Statistik** |
|---|---|---|---|---|
| Urin von Blasenkarzinomen | 25 (83%) | 5 | 30 | |
| Urin von benignen urologischen Erkrankungen | 4 (27%) | 11 | 15 | |
| normaler Urin | 3 (15%) | 17 | 20 | |
| Sensitivität | | | | 83% |
| positiver Vorhersagewert | | | | 81% |

Tabelle 3 zeigt einen Vergleich der Ergebnisse aus Publikationen drei verschiedener Autoren, welche Bestimmungen der Telomeraseaktivität in Gewebeproben, Blasenspülungen und Urin aus Patienten mit Harnblasenkarzinomen durchgeführt haben.

**Tabelle 3**

| **Probe** | **Müller et al.** | **Yoshida et al.** | **Kinoshita et al.** |
|---|---|---|---|
| Gewebe | 96% | 86% | 98% |
| Blasenspülung | 73% | - | 84% |
| Urin | 0% | 62% | 55% |

Ein Vergleich von Tabelle 2 und Tabelle 3 zeigt, daß das erfindungsgemäße Verfahren anderen nichtinvasiven Verfahren des Standes der Technik signifikant überlegen ist.

Weitere Ergebnisse, die beim Nachweis von hTR erhalten wurden, sind in den folgenden Tabellen 4 und 5 dargestellt. Tabelle 4 zeigt Ergebnisse beim Nachweis von hTR bei Patienten mit gesichertem Harnblasenkarzinom in Gewebeproben, Blasenspülungen und Urinproben.

Tabelle 5 zeigt Ergebnisse beim Nachweis von hTR in Urinproben von Patienten mit gesichertem Harnblasenkarzinom, benignen urologischen Erkrankungen (Urolithiasis, Harnwegsinfekte) und gesunden Probanden.

**Tabelle 4**

| **Probe** | **hTR positive Proben** | **hTR negative Proben** | **Gesamt** |
|---|---|---|---|
| Gewebe | 27 (96%) | 1 | 28 |
| Blasenspülung | 25 (89%) | 3 | 28 |
| Urin | 23 (82%) | 5 | 28 |

**Tabelle 5**

| **Probe** | **hTR positive Proben** | **hTR negative Proben** | **Gesamt** |
|---|---|---|---|
| Urine bei Harnblasenkarzinom | 36 (80%) | 9 | 45 |
| Urine bei benignen Erkrankungen | 2 (22%) | 7 | 9 |
| Normale Urine | 2 (7%) | 26 | 28 |

Auch diese Ergebnisse bestätigen die hohe Sensitivität und den hohen positiven Vorhersagewert des erfindungsgemäßen Verfahrens.

### Beispiel 2

Nach Zugabe von 10¹ bis 10⁶ Telomerase-positiven J82-Zellen zu 50 ml Urin wurden die Zellen wie in Beispiel 1 beschrieben isoliert und mittels RT-PCR auf das Vorhandensein der humanen Telomerase-RNA-Komponente untersucht. Bei 10⁴ und mehr Zellen pro 50 ml Urin konnten eindeutig positive Ergebnisse erzielt werden.

In einem Parallelansatz wurden die gespikten Urinproben bei 4°C gelagert und anschließend wie in Beispiel 1 beschrieben aufgearbeitet und mittels RT-PCR auf das Vorhandensein der humanen Telomerase-RNA-Komponente untersucht. Nach 48 h Lagerung konnten auch hier bei 10⁴ und mehr Zellen pro 50 ml Urin eindeutig positive Ergebnisse erzielt werden.

Ebenso konnte in Urinproben von Blasenkarzinompatienten noch nach 3-tägiger Lagerung bei 4°C oder Raumtemperatur und anschließender Aufarbeitung wie oben beschrieben die Telomerase-RNA-Komponente eindeutig nachgewiesen werden.

## Patentansprüche

1. Verfahren zum Nachweis von Harnblasenkarzinom in einer Urinprobe,
**dadurch gekennzeichnet,**
**dass** man
(a) eine Urinprobe bereitstellt und
(b) die Telomerase-RNA-Komponente in der Probe bestimmt,
wobei das Vorhandensein der Telomerase-RNA-Komponente in der Probe ein Indikator für das Vorliegen eines Harnblasenkarzinoms ist.

2. Verfahren nach Anspruch 1,
**dadurch gekennzeichnet,**
**dass** Schritt (a) die Isolierung von Zellen aus der Urinprobe und deren aufschluß umfasst.

3. Verfahren nach Anspruch 1 oder 2,
**dadurch gekennzeichnet,**
**dass** die Bestimmung der Telomerase-RNA-Komponente über nichtradioaktive Markierungsgruppen erfolgt, die ausgewählt werden aus immunologisch reaktiven Gruppen, Enzymen, fluoreszierenden Gruppen, lumineszenten Gruppen, Donor-Akzeptor-Systemen, NMRaktiven Markierungsgruppen oder elektronendichten Gruppen.

4. Verfahren nach einem der vorhergehenden Ansprüche,
**dadurch gekennzeichnet,**
**dass** die Bestimmung der Telomerase-RNA-Komponente einen Nukleinsäure-Amplifikationsschritt umfasst.

5. Verfahren nach einem der vorhergehenden Ansprüche,
**dadurch gekennzeichnet,**
**dass** die Bestimmung der Telomerase-RNA-Komponente einen Reversen Transkriptionschritt umfasst.

6. Verfahren nach einem der Ansprüche 4 bis 5,
**dadurch gekennzeichnet,**
**dass** man bei der Bestimmung einer humanen Telomerase-RNA-Komponente für die Nukleinsäure-Amplifikation ein Primerpaar verwendet, ausgewählt aus:
(a) Nukleotiden mit den in SEQ ID NO. 1 und SEQ ID NO. 2 gezeigten Nukleotidsequenzen und
(b) Nukleotiden, die eine Teilsequenz der in SEQ ID NO. 1 und SEQ ID NO. 2 gezeigten Nukleotidsequenzen mit einer Länge von mindestens 5 Nukleotiden enthalten.

7. Verfahren nach einem der Ansprüche 4 bis 6,
**dadurch gekennzeichnet,**
**dass** das Amplifikationsprodukt ohne Auftrennung des Reaktionsansatzes analysiert wird.

8. Verfahren nach Anspruch 7,
**dadurch gekennzeichnet,**
das das Amplifikationsprodukt an einer Festphase immobilisiert wird.

9. Verfahren nach Anspruch 7,
**dadurch gekennzeichnet,**
**dass** das Amplifikationsprodukt durch on-line-Detektion in einem homogenen Testformat bestimmt wird.

10. Verfahren nach einem der vorhergehenden Ansprüche,
**dadurch gekennzeichnet,**
**dass** man neben der Telomerase-RNA-Komponente in der Probe eine davon verschiedene Standard-Nukleinsäure als Standard bestimmt.

11. Verfahren nach Anspruch 10,
**dadurch gekennzeichnet,**
**dass** man bei der Bestimmung von β2-Mikroglobulin-RNA als humaner Standard-RNA ein Primerpaar verwendet, ausgewählt aus:
(a) Nukleotiden mit den in SEQ ID NO. 3 und SEQ ID NO. 4 gezeigten Nukleotidsequenzen und
(b) Nukleotiden, die eine Teilsequenz der in SEQ ID NO. 3 und SEQ ID NO. 4 gezeigten Nukleotidsequenzen mit einer Länge von mindestens 5 Nukleotiden enthalten.

12. Verfahren nach einem der vorhergehenden Ansprüche,
**dadurch gekennzeichnet,**
**dass** man die Bestimmung als Ein-Topf-Reaktion durchführt.

13. Verfahren nach einem der vorhergehenden Ansprüche,
**dadurch gekennzeichnet,**
**dass** man den Nachweis der Telomerase-RNA-Komponente und gegebenenfalls der Standard-RNA unter solchen Bedingungen durchführt, die eine spezifische Detektion der Telomerase-RNA Komponente und - sofern vorhanden - der Standard-RNA ohne Abtrennung von Amplifikationsnebenprodukten erlauben.

14. Verwendung eines Reagenzienkits zum Nachweis der Telomerase-RNA-Komponente, umfassend:
(a) einen Primer für die Reverse Transkription der Telomerase-RNA Komponente,
(b) Mittel zur Reversen Transkription,
(c) ein Primerpaar zur Amplifikation einer durch Reverse Transkription aus der Telomerase-RNA-Komponente erhaltenen DNA,
(d) Mittel zur Nukleinsäure-Amplifikation und
(e) Markierungsgruppen
in einem nichtinvasiven Verfahren zum Nachweis von Harnblasenkarzinom.

15. Verwendung nach Anspruch 14 in einem Verfahren nach einem der Ansprüche 1 bis 13.

## Claims

1. Method for the detection of urinary bladder carcinoma in a urine sample,
**characterized in that**
(a) a urine sample is prepared and
(b) the telomerase RNA component is determined in the sample, the presence of the telomerase RNA component in the sample being an indicator for the presence of a urinary bladder carcinoma.

2. Method as claimed in claim 1 or 2,
**characterized in that**
step (a) comprises isolating the cells from the urine sample and lysing them.

3. Method as claimed in claim 1 or 2,
**characterized in that**
the telomerase RNA component is determined by means of non-radioactive marker groups which are selected from immunologically reactive groups, enzymes, fluorescent groups, luminescent groups, donot-acceptor systems, NMR-active marker groups or electron-dense groups.

4. Method as claimed in one of the previous claims,
**characterized in that**
the determination of the telomerase RNA component comprises a nucleic acid amplification step.

5. Method as claimed in one of the previous claims,
**characterized in that**
the determination of the telomerase RNA component comprises a reverse transcription step.

6. Method as claimed in one of the claims 4 to 5,
**characterized in that**
a primer pair is used for the nucleic acid amplification in the determination of a human telomerase RNA component which is selected from:
(a) nucleotides with the nucleotide sequences shown in SEQ ID NO. 1 and SEQ ID NO.2 and
(b) nucleotides which contain a partial sequence of the nucleotide sequences shown in SEQ ID NO. 1 and SEQ ID NO.2 having a length of at least 5 nucleotides.

7. Method as claimed in one of the claims 4 to 6,
**characterized in that**
the amplification product is analysed without separating the reaction mixture.

8. Method as claimed in claim 7,
**characterized in that**
the amplification product is immobilized on a solid phase.

9. Method as claimed in claim 7,
**characterized in that**
the amplification product is determined by on-line detection in a homogeneous test format.

10. Method as claimed in one of the previous claims,
**characterized in that**
in addition to the telomerase RNA component in the sample, a standard nucleic acid that differs therefrom is determined as a standard.

11. Method as claimed in claim 10,
**characterized in that**
a primer pair is used for the determination of β2-microglobulin RNA as a human standard RNA which is selected from:
(a) nucleotides with the nucleotide sequences shown in SEQ ID NO. 3 and SEQ ID NO. 4 and
(b) nucleotides which contain a partial sequence of the nucleotide sequences shown in SEQ ID NO.3 and SEQ ID NO.4 with a length of at least 5 nucleotides.

12. Method as claimed in one of the previous claims,
**characterized in that**
the determination is carried out as a one-pot reaction.

13. Method as claimed in one of the previous claims,
**characterized in that**
the telomerase RNA and optionally the standard RNA are detected under conditions that allow a specific detection of the telomerase RNA component and - if present - the standard nucleic acid without separation of amplification by-products.

14. Reagent kit for the detection of the telomerase RNA component comprising:
(a) a primer for the reverse transcription of the telomerase RNA component
(b) an agent for reverse transcription,
(c) a primer pair for the amplification of a DNA obtained from the telomerase RNA component by reverse transcription,
(d) an agent for nucleic acid amplification and
(e) marker groups
in a non-invasive method for detecting urinary bladder carcinoma.

15. Use as claimed in claim 14 in a method as claimed in one of the claims 1 to 13.

## Revendications

1. Procédé de détection d'un carcinome de la vessie dans un échantillon d'urine, **caractérisé en ce que**
(a) on prépare un échantillon d'urine et
(b) on détermine la composante ARN de la télomérase dans l'échantillon, la présence de la composante ARN de la télomérase dans l'échantillon étant un indicateur de l'existence d'un carcinome de la vessie.

2. Procédé selon la revendication 1, **caractérisé en ce que** l'étape (a) comprend l'isolement de cellules de l'échantillon d'urine et leur désagrégation.

3. Procédé selon la revendication 1 ou 2, **caractérisé en ce que** la détermination de la composante ARN de la télomérase s'effectue par l'intermédiaire de groupes marqueurs non radioactifs, qui sont choisis parmi des groupes immunologiquement réactifs, des enzymes, des groupes fluorescents, des groupes luminescents, des systèmes donneur-accepteur, des groupes marqueurs actifs en RMN ou des groupes à forte densité électronique.

4. Procédé selon l'une des revendications précédentes, **caractérisé en ce que** la détermination de la composante ARN de la télomérase comprend une étape d'amplification d'acides nucléiques.

5. Procédé selon l'une des revendications précédentes, **caractérisé en ce que** la détermination de la composante ARN de la télomérase comprend une étape de transcription inverse.

6. Procédé selon l'une des revendications 4 à 5, **caractérisé en ce que**, lors de la détermination d'une composante ARN de la télomérase humaine, on utilise une paire d'amorces choisies parmi:
(a) des nucléotides ayant les séquences de nucléotides représentées par SEQ ID N° 1 et SEQ ID N° 2 et
(b) des nucléotides contenant une séquence partielle, d'une longueur d'au moins 5 nucléotides, des séquences de nucléotides représentées par SEQ ID N° 1 et SEQ ID N° 2.

7. Procédé selon l'une des revendications 4 à 6, **caractérisé en ce que** l'on analyse le produit d'amplification sans séparation du mélange réactionnel.

8. Procédé selon la revendication 7, **caractérisé en ce que** le produit d'amplification est immobilisé sur une phase solide.

9. Procédé selon la revendication 7, **caractérisé en ce que** l'on détermine le produit d'amplification par une détection en ligne dans un format d'essai homogène.

10. Procédé selon l'une des revendications précédentes, **caractérisé en ce que**, en plus de la composante ARN de la télomérase dans l'échantillon, on détermine en tant que référence un acide nucléique de référence qui en est différent.

11. Procédé selon la revendication 10, **caractérisé en ce que**, dans la détermination d'ARN de β2-microglobuline comme ARN de référence humain, on utilise une paire d'amorces choisies parmi
(a) des nucléotides ayant les séquences de nucléotides représentées par SEQ ID N° 3 et SEQ ID N° 4 et
(b) des nucléotides contenant une séquence partielle, d'une longueur d'au moins 5 nucléotides, des séquences de nucléotides représentées par SEQ ID N° 3 et SEQ ID N° 4.

12. Procédé selon l'une des revendications précédentes, **caractérisé en ce que** l'on effectue la détermination sous forme d'une réaction en un seul récipient.

13. Procédé selon l'une des revendications précédentes, **caractérisé en ce que** l'on effectue la détection de la composante ARN de la télomérase et éventuellement de l'ARN de référence dans des conditions qui permettent une détection spécifique de la composante ARN de la télomérase et - dans la mesure où il est présent - de l'ARN de référence, sans séparation des produits secondaires de l'amplification.

14. Utilisation d'un kit de réactifs pour la détermination de la composante ARN de la télomérase, comprenant:
(a) une amorce pour la transcription inverse de la composante ARN de la télomérase,
(b) des agents pour la transcription inverse,
(c) une paire d'amorces pour l'amplification d'un ADN obtenu par transcription inverse de la composante ARN de la télomérase,
(d) des agents pour l'amplification de l'acide nucléique et
(e) des groupes marqueurs
dans un procédé non invasif de détermination du cancer de la vessie.

15. Utilisation selon la revendication 14 dans un procédé selon l'une des revendications 1 à 13.
